# EUROPEAN PATENT APPLICATION

(11) **EP 3 812 786 A1**
(43) Date of publication of application: **28.04.2021**
(21) Application number: 19205277.7
(22) Date of filing: 25.10.2019
(51) Int. Cl.: G01R 33/565, G01R 33/385

(54) **MAGNETIC RESONANCE IMAGING SYSTEM WITH INTENTIONAL GRADIENT NOISE**

(71) Applicant: Koninklijke Philips N.V., 5656 AG Eindhoven (NL)
(72) Inventor: WEISS, Steffen, 5656 AE Eindhoven (NL); HELLE, Michael Günter, 5656 AE Eindhoven (NL); BUSSA, Nagaraju, 5656 AE Eindhoven (NL); JOHNSON, Mark Thomas, 5656 AE Eindhoven (NL); SISODIA, Rajendra Singh, 5656 AE Eindhoven (NL)
(74) Representative: Philips Intellectual Property & Standards

(57) **Abstract**

The present invention relates to a magnetic resonance imaging system (10), comprising at least one gradient coil (20), and a processing unit (30). The processing unit is configured to control a gradient coil to produce intentional noise for sedation monitoring of a patient.

## Description

### FIELD OF THE INVENTION

The present invention relates to a magnetic resonance imaging system, a method of sedation monitoring of a patient, as well as to a computer program element and a computer readable medium.

### BACKGROUND OF THE INVENTION

Interaction with patients during medical imaging with a Magnetic Resonance image (MRI) acquisition unit or system is challenging. In a considerable fraction of MR imaging sessions, patients are subjected to sedation to alleviate uncomfortably long scan times, to relief anxiety, or to minimize voluntary patient motion and thus increase image quality. For example, strong MR noise may cause patients to move or at least cause discomfort, if patients are only lightly sedated. Such motions frequently compromise image quality, and sedation may be even required in order to perform certain types of scans that are very motion-sensitive. Sedation is currently administered manually by medical staff, and sedation depth is usually monitored by clinical tests as responsiveness to verbal and tactile stimulation. The identification of the objective sedation status is one of the key problems for the imaging to select the right protocol with an adapted timing sequence and also to identify how the sedation status is changing to decide on next steps.

It is particularly challenging to assess the sedation state in the noisy environment of a scanner system. Standard MR imaging sessions are performed under full control of experienced staff members. They position the patient on the MR patient support, apply sensors such as an ECG unit for cardiac scans, select, adapt and run the specific set of scans, perform some immediate image quality control, archive the images, and dismiss the patient from the MR suite. Because MR sessions are typically relatively long, this represents a large cost factor in radiology. Therefore, more autonomous imaging approaches are desirable, in which at least some of the above steps are automatized. Other drivers towards autonomous imaging are generally the increasing numbers of MR examinations and a lack of experienced staff in many regions. Various stimuli have been proposed to measure sedation depth automatically (visual, audio, taste, haptics) in an autonomous imaging setting. However, these additional devices need to be integrated with the scanner, which causes additional cost of the system. This is because any device integrated with the MR system needs to be adapted to avoid any electromagnetic interference with the MR system, and the device must not emit any RF that would compromise MR image quality. It must not contain any ferromagnetic components, and any electrical wiring must be carefully designed to avoid undesirable heating or loss of image quality. Additionally, the device must be designed such that the high static and time-varying electric and magnetic fields of the MR system do not compromise the safety or function of the device. All these tasks require considerable design effort, safety testing, and are subject to medical regulation.

There is a need to address these issues.

### SUMMARY OF THE INVENTION

It would be advantageous to have improved means of sedation monitoring a patient undergoing a medical scan with an MRI system. The object of the present invention is solved with the subject matter of the independent claims, wherein further embodiments are incorporated in the dependent claims. It should be noted that the following described aspects and examples of the invention apply also to the magnetic resonance imaging system, the method of sedation monitoring of a patient, as well as to the computer program element and a computer readable medium.

In a first aspect, there is provided a magnetic resonance imaging system, comprising:
- at least one gradient coil; and
- a processing unit.

The processing unit is configured to control a gradient coil to produce intentional noise for sedation monitoring of a patient.

In other words, an MRI scanner itself is used to produce noise that is used for sedation monitoring of a patient. This means that additional stimulation systems, that have been proposed to be used as part of sedation monitoring and that must be compatible with an MRI system, are not required, or medical staff are not required to manually monitor sedation depth, frequently done via clinical tests. Thus an efficient technique is provided for monitoring the sedation state of the patient is provided, which enables the scan sequence to progress in a manner where the sedation state of the patient can be determined to be appropriate for that particular scan sequence. In this manner, motion artifacts that could be caused due to a patient moving can be mitigated because the patient's sedation state can be monitored in a convenient manner.

In an example, the processing unit is configured to control a waveform of a current applied to the gradient coil to produce the intentional noise for the sedation monitoring of the patient.

In an example, the magnetic resonance imaging system comprises at least one gradient coil drive amplifier. The processing unit is configured to control a gradient coil drive amplifier to drive the gradient coil to produce the intentional noise for the sedation monitoring of the patient.

Thus, one or more gradient coil drive amplifiers can be controlled to provide the intentional noise.

In an example, control of the gradient coil to produce intentional noise comprises utilization by the processing unit of audio relevant information about the patient.

In an example, the magnetic resonance imaging system is configured to acquire at least one patient response to the intentional noise for the sedation monitoring of the patient. The processing unit is configured to determine a sedation state of the patient comprising utilization of the at least one patient response to the intentional noise for the sedation monitoring of the patient.

In an example, the magnetic resonance imaging system comprises at least one sensor device. The at least one sensor device is configured to acquire the at least one patient response to the intentional noise for the sedation monitoring of the patient.

In an example, the processing unit is configured to control MR imaging such that image data acquired by the magnetic resonance imaging system is useable to determine the at least one patient response to the intentional noise for the sedation monitoring of the patient.

Thus, rather than having to use external sensors to monitor the patient, the MRI system can both provide intentional noises for sedation monitoring and then provide image data from a specific scan sequence to determine the sedation state, such as acquiring image data of the face to monitoring facial movements in response to the intentional noise.

In an example, the processing unit is configured to control and/or modify and/or alter a scan sequence on the basis of the determined sedation state of the patient. In this manner, it can be ensured that the patient is at the required sedation state for the scan sequence. Thus, if a patient's sedation state is starting to become lighter a particularly loud scan sequence can be delayed until the patient receives further sedation for example. Rather, and less loud and intrusive part of the scan sequence can progress commensurate with the patient's sedation state, maximising the efficiency of the scanning, and minimising the amount of sedative required to be given to the patient.

In an example, production of the intentional noise for sedation monitoring of a patient comprises the processing unit being configured to produce intentional noise in addition to a magnetic resonance imaging sequence.

In an example, production of the intentional noise for sedation monitoring of a patient comprises the processing unit being configured to modify a magnetic resonance imaging sequence.

In an example, modification of the magnetic resonance imaging sequence comprises an interruption of sequence steps.

In an example, production of the intentional noise for sedation monitoring of a patient comprises the processing unit being configured to re-order steps of a magnetic resonance imaging sequence.

In an example, production of the intentional noise for sedation monitoring of a patient comprises utilization of a particularly loud scan sequence or part of a scan sequence.

In a second aspect, there is provided a method of sedation monitoring of a patient, comprising:
controlling by a processing unit a gradient coil of a magnetic resonance imaging system to produce intentional noise for sedation monitoring of a patient.

According to another aspect, there is provided a computer program element controlling one or more of the apparatuses as previously described which, if the computer program element is executed by a processing unit, is adapted to perform one or more of the methods as previously described.

According to another aspect, there is provided a computer readable medium having stored computer element as previously described.

The computer program element can for example be a software program but can also be a FPGA, a PLD or any other appropriate digital means.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawing:

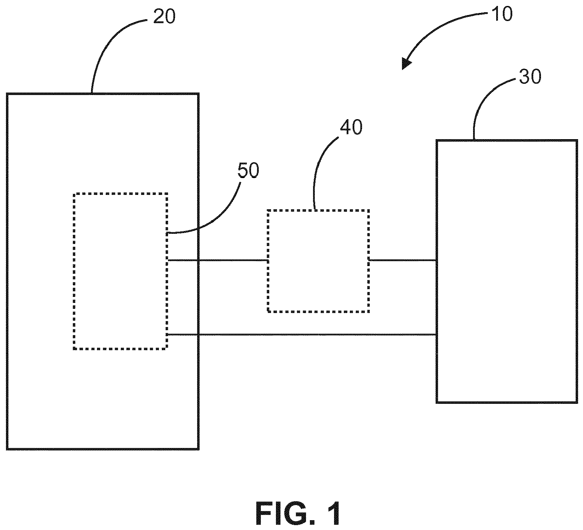

Fig. 1 shows a schematic set up of an example of magnetic resonance imaging system.

### DETAILED DESCRIPTION OF EMBODIMENTS

Fig. 1 shows an example of a magnetic resonance imaging system 10. The system 10 comprises at least one gradient coil 20, and a processing unit 30. The processing unit is configured to control a gradient coil to produce intentional noise for sedation monitoring of a patient.

In an example, the intentional noise comprises music.

In an example, the intentional noise comprises a synthetic representation of a human voice.

In an example, the intentional noise comprises clicks.

In an example, the intentional noise comprises noise having one or more specific pitches.

In an example, the intentional noise comprises noise having one or more specific tones.

In an example, the intentional noise comprises noise having one or more specific durations.

In an example, the intentional noise comprises one or more beeps.

In an example, the intentional noise comprises noise having one or more specific degrees of loudness.

According to an example, the processing unit is configured to control a waveform of a current applied to the gradient coil to produce the intentional noise for the sedation monitoring of the patient.

In an example, the applied current has a high maximum current amplitude.

In an example, the waveform comprises a bipolar trapezoidal gradient waveform.

According to an example, the magnetic resonance imaging system comprises at least one gradient coil drive amplifier 40. The processing unit is configured to control one or more gradient coil drive amplifiers to drive one or more gradient coils to produce the intentional noise for the sedation monitoring of the patient.

According to an example, control of the gradient coil to produce intentional noise comprises utilization by the processing unit of audio relevant information about the patient.

In an example, the audio relevant information comprises an indication of a hearing capability of the patient.

In an example, the audio relevant information comprises information on hearing habits of the patient.

In an example, the audio relevant information comprises information on preferred types of music of the patient.

According to an example, the magnetic resonance imaging system is configured to acquire at least one patient response to the intentional noise for the sedation monitoring of the patient. The processing unit is configured to determine a sedation state of the patient comprising utilization of the at least one patient response to the intentional noise for the sedation monitoring of the patient.

In an example, the apparatus comprises an output unit configured to output the sedation state of the sedated patient.

In other words, a sedation level determination system is provided that can determine the sedation level of a patient for utilization in for example a medical scan procedure.

In an example, output unit can be used to adapt a medical scan procedure based on the responses.

According to an example, the magnetic resonance imaging system comprises at least one sensor device 50. The at least one sensor device is configured to acquire the at least one patient response to the intentional noise for the sedation monitoring of the patient.

In an example, the at least one sensor device comprises: a camera, an EMG sensor, a movement sensor, a tilt sensor, an accelerometer, a microphone, and the at least one sensor device can be the magnetic resonance image acquisition unit itself when operating in an image acquisition mode.

In an example, the at least one patient response comprises: face expression or expressions, eye blinks, body part movement.

According to an example, the processing unit is configured to control MR imaging such that image data acquired by the magnetic resonance imaging system is useable to determine the at least one patient response to the intentional noise for the sedation monitoring of the patient.

According to an example, the processing unit is configured to control and/or modify and/or alter a scan sequence on the basis of the determined sedation state of the patient.

According to an example, production of the intentional noise for sedation monitoring of a patient comprises the processing unit being configured to produce intentional noise in addition to a magnetic resonance imaging sequence.

According to an example, production of the intentional noise for sedation monitoring of a patient comprises the processing unit being configured to modify a magnetic resonance imaging sequence.

According to an example, modification of the magnetic resonance imaging sequence comprises an interruption of sequence steps.

In an example, sequence steps are interrupted in a rhythmic manner.

According to an example, production of the intentional noise for sedation monitoring of a patient comprises the processing unit being configured to re-order steps of a magnetic resonance imaging sequence.

According to an example, production of the intentional noise for sedation monitoring of a patient comprises the processing unit being configured to utilize a particularly loud scan sequence or part of a scan sequence.

From the above description, it is clear that the MRI system can be utilized in a method of sedation monitoring of a patient, where this method comprises controlling by a processing unit a gradient coil of the magnetic resonance imaging system to produce intentional noise for sedation monitoring of the patient.

In an example, the method comprises controlling by the processing unit control a waveform of a current applied to the gradient coil to produce the intentional noise for the sedation monitoring of the patient.

In an example, the magnetic resonance imaging system comprises at least one gradient coil drive amplifier. The method can then comprise controlling by the processing unit a gradient coil drive amplifier to drive the gradient coil to produce the intentional noise for the sedation monitoring of the patient.

In an example, controlling of the gradient coil to produce intentional noise comprises the processing unit utilizing audio relevant information about the patient.

In an example, the method comprises acquiring by the magnetic resonance imaging system at least one patient response to the intentional noise for the sedation monitoring of the patient. The method can then comprise determining by the processing unit a sedation state of the patient utilizing the at least one patient response to the intentional noise for the sedation monitoring of the patient.

In an example, the magnetic resonance imaging system comprises at least one sensor device. The method can then comprise using the at least one sensor device to acquire the at least one patient response to the intentional noise for the sedation monitoring of the patient.

In an example, the method comprises controlling by the processing unit MR imaging such that image data acquired by the magnetic resonance imaging system is useable to determine the at least one patient response to the intentional noise for the sedation monitoring of the patient.

In an example, the method comprises controlling and/or modifying and/or altering by the processing unit a scan sequence on the basis of the determined sedation state of the patient.

In an example, producing the intentional noise for sedation monitoring of a patient comprises the processing unit controlling the gradient coil to produce intentional noise in addition to a magnetic resonance imaging sequence.

In an example, producing the intentional noise for sedation monitoring of a patient comprises the processing unit controlling the gradient coil to modify a magnetic resonance imaging sequence.

In an example, modifying the magnetic resonance imaging sequence comprises interrupting sequence steps.

In an example, producing the intentional noise for sedation monitoring of a patient comprises the processing unit controlling the gradient coil to re-order steps of a magnetic resonance imaging sequence.

In an example, producing the intentional noise for sedation monitoring of a patient comprises the processing unit controlling the gradient coil to produce a particularly loud scan sequence or part of a scan sequence.

The following provides details relating to specific embodiments, and how an understanding of operational characteristics of MRI systems led to a development of such MRI systems to provide a new technique for sedation monitoring, making use of intentional noise generated by the MRI system itself.

MRI patients are routinely subjected to high sound pressure levels, averaging around 95-105 dB, for some MR pulse sequences increasing up to 130 dB. See for example Moelker. A. et al. Importance of Bone-Conducted Sound Transmission on Patient Hearing in the MR Scanner. J Magn Reson Imag 2005; 22:163-169. The noise is due to strong time-varying currents in the gradient coils required to realize the spatial resolution of MR imaging. Lorentz forces on these coils due to the main magnetic field produce the noise. Specifically, the waveform of the currents in the gradient coils (slew rate, maximum strength, shape) in combination with a characteristic scanner-specific transfer function directly determine the pitch, sound, and the noise level. Fast sequences usually require fast switching of gradients, thus, result in high noise levels. In general, MRI scanner noise depends on and varies considerably with the type of scan sequence (TSE, FFE, EPI, Spiral, Cardiac triggered sequences, DWI).

It was realised by the inventors that the noise produced by the gradient coils of the MRI system could itself be used for sedation monitoring. Standard MRI systems have three independent gradient coils X,Y,Z, and each coil consists of several coil parts connected in series so that all parts carry the same current are driven by one of the gradient coil amplifiers X,Y,Z. The inventors realised that the noise produced by these coils could be intentionally utilized for sedation monitoring a patient during an MRI scan, where parts of an MRI image acquisition unit could be utilized and with modifications further beneficial effects could be provided.

Therefore, the MR gradient systems have high fidelity amplifiers that can produce arbitrary current waveforms to be applied to the gradient coils. These waveforms are defined in the MR scan software, and the gradient coils can be driven to produce arbitrary sounds, and even to essentially use the MR gradients as a loudspeaker. Thus, these generated custom sounds are adapted to be useful as an audio stimulus for the measurement of sedation depth. Examples are short and loud beeps, or even a human voice addressing the patient. Frequencies may be used for the beeps where the human ear is particularly sensitive.

It was also realised by the inventors, that the MR scans can be modified such that they provide clearly hearable and characteristic sounds that gain the attention of a patient. For this purpose, the continuous pattern of noise of a standard sequence may be interrupted in a characteristic, e.g. rhythmic way as part of sedation monitoring. Additionally, because standard sequences vary considerably with respect to the loudness of MR noise during different parts of the scan, it was realised that different parts of a scan could be reordered as necessary, such that a particularly loud scans or parts of scans can be used as audio stimulus at an appropriate time point for example, without any modification of their waveforms.

With respect to the generation of intentional noise, the strong currents that are applied to the MR gradient coils to induce noise for sedation monitoring, such as a beep or beeps or synthetic human voice, can have waveforms of currents are interleaved with the waveforms of the scan sequence used for MRI.

Fig. 2 shows a schematic representation of the gradient coils for an MR imaging acquisition unit or scanner. Every gradient coil and coil part produces a characteristic magnetic field distribution that results from the spatial arrangement of the coil leads. These gradient coils (X,Y, or Z) can be used alone, or in combination, to produce the required noise for sedation monitoring.

The response of the patient to the intentional noise can be measured by a number of known methods. Examples are face expressions /eye blinks or other reflexes observed by cameras or sensors, or patient motion can be measured by suitable sensors or can be measured by the MRI system itself.

It is known that patient's hearing capabilities and responses to various types of audio stimuli varies considerably. Thus, the type of stimulus and its loudness to the patient can take into account the specific patient undergoing examination. In this respect, prior knowledge of hearing capabilities / disabilities and other information such as age, hearing habits, preferred types of music can be made use of.

Having monitored the patient, a known sedation depth, determined from the patient's response to the intentional noise, can be utilized to adapt the MR examination to avoid image artefacts. Specifically, the noise characteristics of MR imaging sequences can be changed, or at least the order in which they are executed during an MR examination may be changed. This is done such that patients who are awake or who are only mildly sedated are not subjected to very loud scans, which may cause patient motion and result in image artefacts. The adaptation of the MR sequence based on the sedation depth to avoid image artefacts can proceed as follows. Based on the progression of the sedation depth (shallow), the MR sequence can be adapted for a scan, such that noise stimulus is avoided to a patient who is awake in order not to disrupt the scan. This means that "identifying" specific noise level and converting into corresponding stimulus proportionate to the sedation depth level can be performed in such a way that stimulus is always lower for a specific sedation depth level. For example, if N1 level of noise is a stimulant for sedation depth D1, where a person will not wake-up for N1 noise. Then all the sequences that generates noise less than N1 level can be used.

To augment the monitoring of sedation of a patient, the intentional noise produced by the MRI system can form part of a multi-modal stimuli system that is used to monitor sedation of a patient. Thus, the MR noise can form one sensory stimulus, that can be combined with other sensory stimuli in order to provide a more reliable mechanism to determine the sedation state of the patient. The different stimuli can be provided simultaneously, to provide for an improved ability to determine the sedation state of the patient from how they respond to such congruent multimode stimuli. The different modes of stimuli can be applied in a similar manner, for example having similar or the same frequency waveforms, amplitude waveforms, and can target different sensory organs of the patient. Thus, the MRI system can generate intentional noise, and other stimulation devices are configured to provide stimuli including: mechanical haptic stimuli; TENS stimuli; visual stimuli; nerve and/or muscle stimuli via the Magnetic Resonance Imaging unit itself; questions posed via synthetic generated as part of the "noise" from the MRI system; taste stimuli; fragrance stimuli; balance related stimuli (rocking of the head for example). The sensors used to monitor the multi-modal stimulation can be the same as that used to monitor the patient who is being stimulated just be the MRI system intentionally generated noise. Thus, sensor device can includes one or more of: a camera, an EMG sensor, a movement sensor, a tilt sensor, an accelerometer, a microphone, and for the nerve and/or the Magnetic Resonance Imaging unit/system itself.

In another exemplary embodiment, a computer program or computer program element is provided that is characterized by being configured to execute the method steps of the method according to one of the preceding embodiments, on an appropriate system.

The computer program element might therefore be stored on a computer unit, which might also be part of an embodiment. This computing unit may be configured to perform or induce performing of the steps of the method described above. Moreover, it may be configured to operate the components of the above described apparatus and/or system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A magnetic resonance imaging system (10), comprising:
- at least one gradient coil (20); and
- a processing unit (30);
wherein, the processing unit is configured to control a gradient coil to produce intentional noise for sedation monitoring of a patient.

2. Magnetic resonance imaging system according to claim 2, wherein the processing unit is configured to control a waveform of a current applied to the gradient coil to produce the intentional noise for the sedation monitoring of the patient.

3. Magnetic resonance imaging system according to any of claims 1-2, wherein the magnetic resonance imaging system comprises at least one gradient coil drive amplifier (40), and wherein the processing unit is configured to control a gradient coil drive amplifier to drive the gradient coil to produce the intentional noise for the sedation monitoring of the patient.

4. Magnetic resonance imaging system according to any of claims 1-3, wherein control of the gradient coil to produce intentional noise comprises utilization by the processing unit of audio relevant information about the patient.

5. Magnetic resonance imaging system according to any of claims 1-4, wherein the magnetic resonance imaging system is configured to acquire at least one patient response to the intentional noise for the sedation monitoring of the patient; and wherein the processing unit is configured to determine a sedation state of the patient comprising utilization of the at least one patient response to the intentional noise for the sedation monitoring of the patient.

6. Magnetic resonance imaging system according to claim 5, wherein the magnetic resonance imaging system comprises at least one sensor device (50); wherein the at least one sensor device is configured to acquire the at least one patient response to the intentional noise for the sedation monitoring of the patient.

7. Magnetic resonance imaging system according to claim 5, wherein the processing unit is configured to control MR imaging such that image data acquired by the magnetic resonance imaging system is useable to determine the at least one patient response to the intentional noise for the sedation monitoring of the patient.

8. Magnetic resonance imaging system according to any of claims 5-7, wherein the processing unit is configured to control and/or modify and/or alter a scan sequence on the basis of the determined sedation state of the patient.

9. Magnetic resonance imaging system according to any of claims 1-8, wherein production of the intentional noise for sedation monitoring of a patient comprises the processing unit being configured to produce intentional noise in addition to a magnetic resonance imaging sequence.

10. Magnetic resonance imaging system according to any of claims 1-9, wherein production of the intentional noise for sedation monitoring of a patient comprises the processing unit being configured to modify a magnetic resonance imaging sequence.

11. Magnetic resonance imaging system according to claim 10, wherein modification of the magnetic resonance imaging sequence comprises an interruption of sequence steps.

12. Magnetic resonance imaging system according to any of claims 1-11, wherein production of the intentional noise for sedation monitoring of a patient comprises the processing unit being configured to re-order steps of a magnetic resonance imaging sequence.

13. Magnetic resonance imaging system according to claim 12, comprising utilization of a particularly loud scan sequence or part of a scan sequence.

14. A method of sedation monitoring of a patient, comprising:
controlling by a processing unit a gradient coil of a magnetic resonance imaging system to produce intentional noise for sedation monitoring of a patient.

15. A computer program element for controlling a system according to any of claims 1-13, which when executed by a processor is configured to carry out the method of claim 14.
